# EUROPÄISCHE PATENTSCHRIFT

(11) **EP 2 414 029 B1**
(45) Veröffentlichungstag und Bekanntmachung des Hinweises auf die Patenterteilung: **21.11.2012**
(21) Anmeldenummer: 10702285.7
(22) Anmeldetag: 29.01.2010
(51) Int. Cl.: A61M 37/00, A61B 17/20, A61K 9/70, A61M 35/00, A61B 10/00

(54) **APPLIKATOR ZUR BEHANDLUNG VON HAUT**
APPLICATOR FOR TREATING SKIN
APPLICATEUR POUR TRAITEMENT DE LA PEAU

(30) Priorität: 31.03.2009 DE 102009002019
(43) Veröffentlichungstag der Anmeldung: 08.02.2012
(73) Patentinhaber: Robert Bosch GmbH, 70442 Stuttgart (DE)
(72) Erfinder: STUMBER, Michael, 70825 Korntal-Muenchingen (DE)
(86) Internationale Anmeldenummer: PCT/EP2010/051089
(87) Internationale Veröffentlichungsnummer: WO 2010/112244

(56) Entgegenhaltungen:
- WO-A1-2008/062032
- WO-A2-99/63934
- DE-A1-102004 032 892
- US-A- 5 402 798
- US-A1- 2007 161 964

## Beschreibung

### Stand der Technik

Die Erfindung betrifft einen Applikator zur Behandlung von Haut gemäß dem Oberbegriff des Anspruchs 1.

Die US 5402798 offenbart eine Vorrichtung zum Durchstechen einer Haut, wobei die Vorrichtung einen Belag aufweist, der mit einem Desinfektionsmittel imprägniert ist.

Ein derartiger Applikator ist aus der DE 10 2004 032 892 A1 bekannt. Der bekannte Applikator weist eine mittels einer Hubeinrichtung betätigbare Nadelplatte auf, die die Haut an der Stelle, an der später beispielsweise ein Wirkstoffpflaster aufgebracht werden soll, mehrmals perforiert, um damit Durchgänge für die im Wirkstoffpflaster gespeicherten Wirkstoffe zu schaffen. Über derartige Wirkstoffpflaster können die Wirkstoffe durch die Haut in den Körper gelangen, solange die Durchgänge geöffnet sind. Durch den Selbstheilungsprozess des Körpers schließen sich dabei die Durchgänge beispielsweise nach einigen Stunden wieder selbstständig. Als Nadeln kommen dabei insbesondere so genannte Mikronadeln zum Einsatz, die bei richtiger Dimensionierung beim Perforieren zumindest als weitgehend schmerzfrei empfunden werden.

Weiterhin sind aus dem Stand der Technik Mikronadeln aus porösem Silizium oder biologisch abbaubarem Polymer bekannt, die in der Haut verbleiben können. Das Material zersetzt sich bei geeigneter Herstellung durch die intrazelluläre Flüssigkeit des Körpers langsam von selbst, so dass mit derartigen Mikronadeln die Durchgänge für den Wirkstoff während ca. 2 bis 3 Tage passierbar sind.

Als problematisch bei der Verwendung eines nach dem Oberbegriff des Anspruchs 1 ausgebildeten Applikators wird angesehen, dass die Anwendung zum Beispiel von Wirkstoffpflastern eine Vorbereitung bzw. Desinfektion der Haut an der zu perforierenden Stelle erfordert, damit Infektionen der perforierten Hautstelle vermieden werden. Dabei erfolgt das Desinfizieren der zu perforierenden Hautstelle üblicherweise manuell zum Beispiel mittels eines mit Desinfektionsmittel getränkten Wattebausches, wobei Fehler sowohl bezüglich der Stelle mit Blick auf die anschließend perforierte Hautstelle, als auch der richtigen Menge an Desinfektionsmittel auftreten können. Ferner ist eine hohe räumliche Genauigkeit auch bei dem Anbringen des Wirkstoffpflasters erforderlich, damit die Wirkstoffe des Wirkstoffpflasters möglichst vollständig in die Haut gelangen können.

### Offenbarung der Erfindung

Der Erfindung liegt daher die Aufgabe zugrunde, einen Applikator zur Behandlung von Haut gemäß dem Oberbegriff des Anspruchs 1 derart weiterzubilden, dass die Handhabung bei der Anwendung zum Beispiel von Wirkstoffpflastern erleichtert und gleichzeitig zumindest die Gefahr von Infektionen verringert wird. Diese Aufgabe wird bei einem Applikator mit den Merkmalen des Anspruchs 1 gelöst. Der Erfindung liegt dabei der Gedanke zugrunde, die Schritte des Desinfizierens und Perforierens der Hautstelle durch ein und denselben Applikator nacheinander auszuführen, ohne dass hierzu ein mehrmaliges Ansetzen des Applikators an die zu behandelnde Hautstelle erforderlich ist. Dadurch wird eine sehr hohe Positionsgenauigkeit bzgl. der unterschiedlichen Behandlungen der Hautstelle erzielt. Das bedeutet, dass ein erfindungsgemäßer Applikator neben der Einrichtung zur Perforation der Hautstelle zumindest auch noch eine Einrichtung zur Desinfektion der Hautstelle aufweist, die über dieselbe Öffnung am Gehäuse auf die zu behandelnde Hautstelle wirkt. Dadurch wird sichergestellt, dass die zu perforierende Hautstelle in der erforderlichen Weise sowohl bezüglich des Ortes der Desinfektion, als auch bezüglich der Menge beziehungsweise der Ausführung der Desinfektion optimal hinsichtlich der anschließenden Perforation vorbereitet wird, wodurch nachträgliche Infektionen vermieden werden können.

Vorteilhafte Weiterbildungen des erfindungsgemäßen Applikators zur Behandlung von Haut sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

In einer bevorzugten Ausbildung des Applikators ist es vorgesehen, dass eine Steuereinrichtung vorgesehen ist, die die Einrichtung zur Perforation und die Einrichtung zur Desinfektion selbstständig entsprechend zumindest eines abgespeicherten Programmablaufs ansteuert und dass Mittel vorgesehen sind, die die Einrichtung zur Perforation und die Einrichtung zur Desinfektion entsprechend des Programmablaufs durch die Öffnung des Gehäuses in Wirkverbindung mit der Hautstelle bringen. Dadurch lässt sich ein vollautomatischer Ablauf der Behandlungsschritte erzielen, so dass eine besonders einfache und fehlerfreie Bedienung des Applikators möglich ist.

In einer weiteren Ausgestaltung der Erfindung ist es vorgesehen, dass zusätzlich eine Einrichtung zum Anbringen eines Wirkstoffpflasters auf die zuvor desinfizierte und perforierte Hautstelle vorgesehen ist, und dass die Einrichtung zum Anbringen eines Wirkstoffpflasters ebenfalls von der Steuereinrichtung automatisch ansteuerbar ist. Mittels dieser Ausgestaltung lassen sich Wirkstoffpflaster positionsgenau und automatisch an die zuvor desinfizierte und perforierte Hautstelle anbringen, so dass auch hier Handhabungsfehler vermieden werden.

Die Desinfektionseinrichtung lässt sich konstruktiv relativ einfach realisieren, wenn diese einen mittels eines Desinfektionsmittels behandelten Träger aufweist, welcher unter Anlagekontakt über die zu desinfizierende Hautstelle bewegbar ist.

Konstruktiv ist es dabei vorteilhafterweise vorgesehen, dass der Träger bandförmig ausgebildet ist, dass der Träger auf seiner in Kontakt mit der Hautstelle gelangenden Außenseite einen saugfähigen Stoff aufweist und, dass ein Vorratsbehälter für das Desinfektionsmittel vorgesehen ist, über den die Außenseite mit Desinfektionsmittel benetzbar ist. Dadurch weist die Desinfektionseinrichtung eine hohe Standzeit auf und arbeitet besonders sicher.

Um bei einem manuellen Anbringen eines Wirkstoffpflasters eine positionsgenaue Positionierung des Wirkstoffpflasters zu ermöglichen, ist es besonders vorteilhaft, wenn eine Markiereinrichtung vorgesehen ist, die die Desinfektionseinrichtung oder die Perforationseinrichtung umfangsseitig umgibt und die in Anlagekontakt mit der Hautstelle bringbar ist, um die Hautstelle bezüglich ihrer Ausdehnung optisch zu kennzeichnen.

Um die Hautstelle zu perforieren ist es darüber hinaus vorgesehen, dass die Perforationseinrichtung einen Perforationsstempel mit einer Vielzahl von nebeneinander angeordneten Mikronadeln aufweist. Ein derartiger Perforationsstempel ist kompakt aufgebaut und ermöglicht eine sichere Perforation der Hautstelle bei hoher Standzeit.

Alternativ bzw. zur Erzielung von Durchgängen in der Hautstelle, die sich erst nach längerer Zeit wieder schließen ist es jedoch in einer Weiterbildung vorgesehen, dass die Perforationseinrichtung eine Vielzahl von Mikronadeln aufweist, die an einem Träger angeordnet sind und, dass die Mikronadeln als selbstauflösende Mikronadeln ausgebildet sind, welche von dem Träger abtrennbar sind. Die Mikronadeln können dabei beispielsweise aus porösem Silizuim oder biologisch abbaubarem Polymer bestehen.

Um zumindest die Desinfektionseinrichtung und die Perforationseinrichtung in den Bereich der Öffnung des Gehäuses zu bringen ist es in einer konstruktiv vorteilhaften Ausgestaltung vorgesehen, dass in dem Gehäuse eine Trägereinrichtung angeordnet ist und, dass mittels der Trägereinrichtung die Perforationseinrichtung und die Desinfektionseinrichtung in den Bereich der Öffnung verschwenkbar sind.

Besonders vorteilhaft ist es darüber hinaus, wenn zumindest die Perforationseinrichtung und die Desinfektionseinrichtung an der Trägereinrichtung auswechselbar angeordnet sind. Dadurch lassen sich am Ende der Standzeit bzw. zum Nachfüllen von Desinfektionsmittel diese Einrichtungen einfach austauschen, so dass der Applikator eine besonders hohe Lebensdauer aufweist bzw. immer wieder verwendet werden kann.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: einen Applikator zur Behandlung von Haut in einem vereinfacht dargestellten Längsschnitt,
- Fig. 2: einen Teilbereich des Applikators nach Fig. 1 beim Desinfizieren einer Hautstelle in einem Längsschnitt,
- Fig. 3: einen Teilbereich des Applikators nach Fig. 1 beim Perforieren der in Fig. 2 desinfizierten Hautstelle in einem Längsschnitt,
- Fig. 4: einen Teilbereich des Applikators nach Fig. 3 nach dem Perforieren der Hautstelle in einem Längsschnitt und
- Fig. 5: die zuvor desinfizierte und perforierte Hautstelle nach dem Anbringen eines Wirkstoffpflasters im Schnitt.

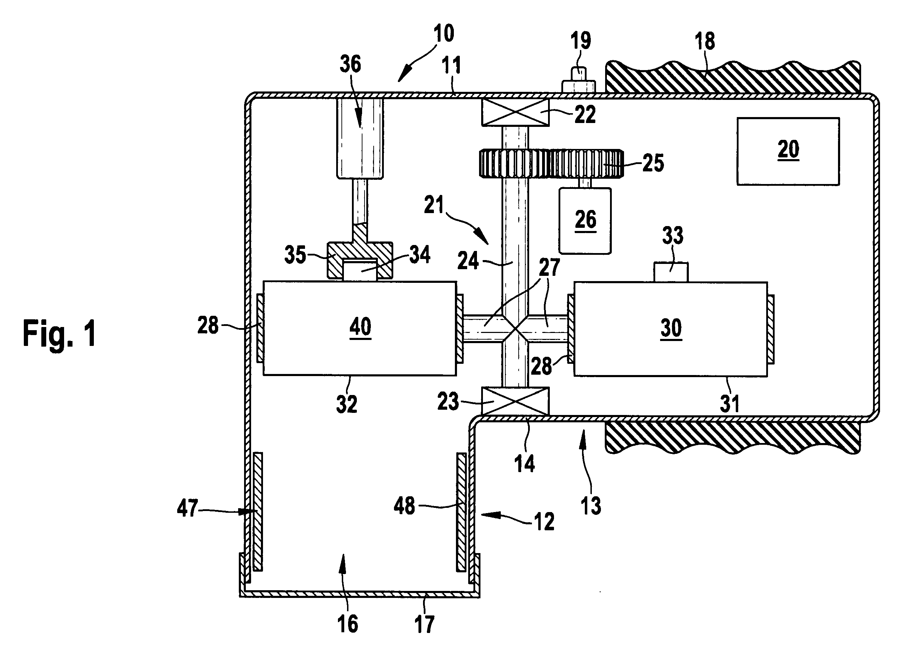

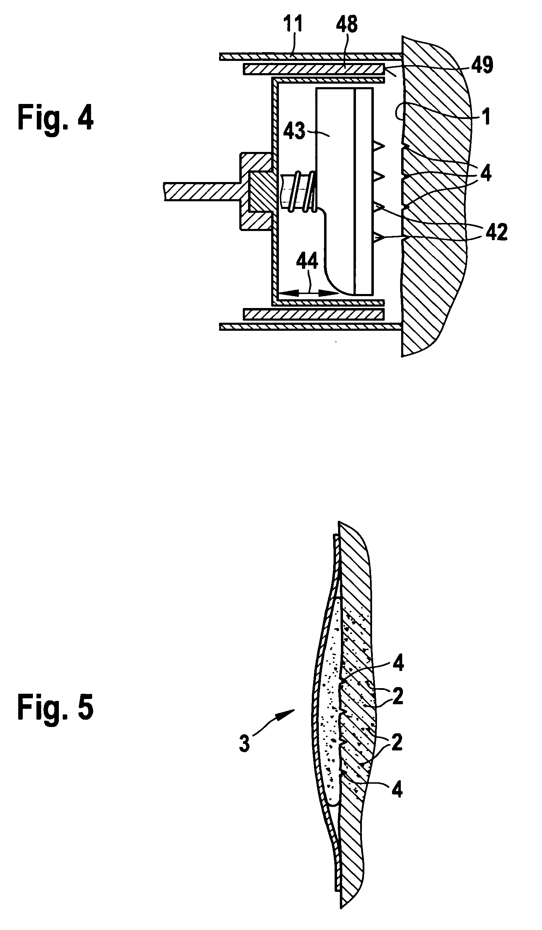

In Fig. 1 ist ein Applikator 10 zur Behandlung einer Hautstelle 1, insbesondere einer Hautstelle 1 eines Menschen dargestellt. Auf die Hautstelle 1 soll ein medizinische Wirkstoffe 2 enthaltendes Wirkstoffpflaster 3 aufgebracht werden, welches seine Wirkstoffe 2 durch in der Hautstelle 1 eingebrachte Durchgänge bzw. Perforationen 4 in den menschlichen Körper abgibt (Fig. 5). Der Applikator 10 dient hierbei zumindest zur Vorbereitung der Hautstelle 1, damit das Wirkstoffpflaster 3 auf die Hautstelle 1 positionsgenau aufgebracht werden kann.

Der Applikator 10 weist ein Gehäuse 11 mit einem ersten, im Querschnitt insbesondere rechteckförmigen oder quadratischen Bereich 12 auf, an dem seitlich ein als Handgriff 13 ausgebildeter zweiter Bereich 14 einstückig angeformt ist, so dass sich im Querschnitt eine in etwa L-förmige Form des Gehäuses 11 ergibt. Das Gehäuse 11 weist auf seiner in Anlagekontakt mit der Hautstelle 1 kommenden Seite eine Öffnung 16 auf, die mittels eines abnehmbaren Schutzdeckels 17 verschließbar ist. An der Außenseite des Handgriffs 13 ist eine anatomisch ausgebildete Anformung 18 als separates Bauteil oder einstückig am Gehäuse 11 angeordnet. Mittels der Anformung 18 kann der Applikator 10 mit einer Hand einer Bedienperson, die identisch mit der zu behandelnden Person sein kann gehalten werden. An der Außenseite des Gehäuses 11 ist weiterhin ein Betätigungsschalter 19 angeordnet, der mit einer im Inneren des Gehäuses 11 angeordneten Steuereinrichtung 20 des Applikators 10 elektrisch verbunden ist.

Im Inneren des Gehäuses 11 weist der Applikator 10 beispielhaft eine Trägereinrichtung 21 auf. Die Trägereinrichtung 21 umfasst eine in zwei Lagern 22, 23 drehbar gelagerte Welle 24, die über ein Zahnrad 25 und einen von der Steuereinrichtung 20 ansteuerbaren Stellmotor 26 drehbar ist. An der Welle 24 sind insbesondere in gleichmäßigen Winkelabständen Trägerarme 27 drehfest angeordnet, an deren äußeren Ende jeweils in einem Halter 28 Funktionseinheiten 30, 40 angeordnet sind.

Die als Einrichtungen zum Desinfizieren bzw. zum Perforieren der Hautstelle 1 ausgebildeten Funktionseinheiten 30, 40 weisen jeweils ein eigenes Gehäuse 31, 32 auf, das an seiner Oberseite einen Adapter 33, 34 hat, der mit einem entsprechend geformten Gegenstück 35 eines Hubmechanismus 36 zusammenwirkt. Mittels des Hubmechanismus 36 ist die entsprechende, von der Steuereinrichtung 20 angesteuerte Funktionseinheit 30, 40 aus dem Halter 28 entnehmbar und bis in den Bereich der Öffnung 16 des Gehäuses 11 verbringbar und umgekehrt. Die Funktionseinheiten 30, 40 dienen jeweils einer bestimmten Behandlung der Hautstelle 1. So dient die erste Funktionseinheit 30 der Desinfektion der Hautstelle 1, während die zweite Funktionseinheit 40 der Perforation der Hautstelle 1 dient.

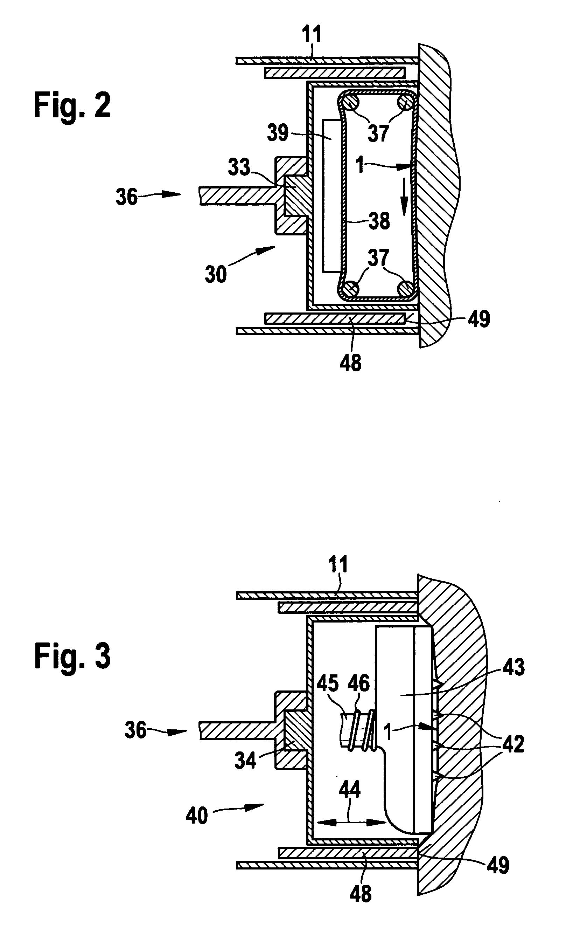

Wie am besten aus Fig. 2 ersichtlich ist, weist die erste Funktionseinheit 30 in ihrem Gehäuse 31 mehrere drehbar gelagerte Rollen 37 auf, wobei mindes-tens eine der Rollen 37 mittels eines nicht dargestellten Antriebs antreibbar ist. Um die Rollen 37 spannt sich ein bandförmiger Träger 38 für ein Desinfektions-mittel. Der Träger 38 besteht zumindest an seiner in Kontakt mit der Hautstelle 1 gelangenden Außenseite aus saugfähigem Stoff, zum Beispiel Fleece, einer Wattierung oder ähnlichem. Auf der der Öffnung 16 gegenüberliegenden Seite des Trägers 38 ist dieser in Wirkverbindung mit einem Vorratsbehälter 39 für das Desinfektionsmittel angeordnet. Bei dem Desinfektionsmittel handelt es sich um ein üblicherweise zur Desinfektion von Haut verwendetes Desinfektionsmittel wie zum Beispiel Ethanol. Über den Vorratsbehälter 39 wird der Träger 38 an seiner zugewandten Seite mit dem Desinfektionsmittel benetzt bzw. getränkt.

Die zweite Funktionseinheit 40 weist, wie aus Fig. 3 ersichtlich ist, einen mit einer Vielzahl von nebeneinander angeordneten Mikronadeln 42 versehenen Perforationsstempel 43 für die Hautstelle 1 auf. Der Perforationsstempel 43 ist innerhalb seines auf der der Öffnung 16 zugewandten Seite offenen Gehäuses 32 entsprechend des Doppelpfeils 44 axial bewegbar. Bei der Anwendung an der Hautstelle 1 bedeutet dies, dass sich der Perforationsstempel 43 im Wesentli-chen senkrecht zur Hautstelle 1 bewegt. Der Perforationsstempel 43 weist einen Schaft 45 auf, der von einer Druckfeder 46 umfasst ist. Mittels der Kraft der Druckfeder 46 wird der Perforationsstempel 43 bzw. dessen Mikronadeln 42 zum Perforieren mit einer definierten Kraft (zum Beispiel, weil die Druckfeder 46 ent-sprechend vorgespannt ist) gegen die Hautstelle 1 gedrückt. Alternativ dazu ist es auch möglich, die Bewegung des Perforationsstempels 43 zur Durchführung der Perforation entweder über den Hubmechanismus 36, oder aber über einen anderen, insbesondere in der Funktionseinheit 40 integral angeordneten geeig-neten Mechanismus auszuführen. Ggf. können auch zusätzliche, beispielsweise optisch arbeitende Sensoren vorgesehen sein, die sicherstellen, dass die Haut-stelle 1 in der richtigen Art und Weise perforiert wurde.

Ferner kann zusätzlich vorgesehen sein, dass innerhalb des Gehäuses 11 im Bereich der Öffnung 16 der Perforationsstempel 43 an seinem Umfang von einer Markiereinrichtung 47 umfasst ist. Die Markiereinrichtung 47 weist einen Markierrahmen 48 auf, dessen der Öffnung 16 zugewandte Stirnfläche 49 mit einer hautverträglichen Farbe behandelt ist, die sich bei Kontakt mit der Hautstelle 1 auf die Hautstelle 1 überträgt. Die Bewegung des Markierrahmens 48 ist vorzugsweise mit dem Perforationsstempel 43 gekoppelt.

Anstelle der Verwendung einer Markiereinrichtung 47 ist es alternativ auch möglich, das Desinfektionsmittel einzufärben, so dass dieses beim Desinfizieren der Hautstelle 1 mittels des Trägers 38 auf die Hautstelle 1 übertragen wird bzw. die desinfizierte Hautstelle 1 farblich kennzeichnet.

Die Arbeitsweise des Applikators 10 wird wie folgt erläutert: In einem ersten Schritt wird der Applikator 10 mit seiner Öffnung 16 von der Bedienperson manuell auf die zu perforierende Hautstelle 1 aufgesetzt. Sobald dies geschehen ist, drückt die Bedienperson den Betätigungsschalter 19. Dies bewirkt in der Steuereinrichtung 20 den Start eines automatisch ablaufenden Programms. Das Programm sieht in einem ersten Programmschritt vor, dass die erste, der Desinfektion der Hautstelle 1 dienende Funktionseinheit 30 angesteuert wird. Diese wird mittels des Hubmechanismus 36 gemäß Fig. 2 in Anlagekontakt mit der Hautstelle 1 gebracht, worauf der mit Desinfektionsmittel getränkte Träger 38 mit-tels der wenigstens einen angetriebenen Rolle 37 in Bewegung versetzt wird, was eine vollflächige Desinfektion der Hautstelle 1 durch Vorbeistreifen des Trä-gers 38 auf der Hautstelle 1 bewirkt. Anschließend werden von der Steuereinrich-tung 20 in einem zweiten Programmschritt die zweite Funktionseinheit 40 mit dem Perforationsstempel 43 sowie der Markiereinrichtung 47 angesteuert. Diese bewirken entsprechend Fig. 3 eine Perforation der Hautstelle 1, so dass die Perforationen 4 innerhalb der vorher desinfizierten Hautstelle 1 ausgebildet wer-den. Gleichzeitig wird der perforierte Bereich mittels der Markiereinrichtung 47 farbig und somit anschließend für die Bedienperson optisch erkennbar gekenn-zeichnet. Sobald dies erfolgt ist, kann die Bedienperson den Applikator 10 von der Hautstelle 1 wieder abnehmen bzw. entfernen (Fig. 4) und in einem letzten Schritt das Wirkstoffpflaster 3 auf die zuvor farblich gekennzeichnete Hautstelle 1 positionsgenau anbringen (Fig. 5). Nunmehr können die Wirkstoffe 2 aus dem Wirkstoffpflaster 3 durch die Perforationen 4 in den Körper gelangen.

Ergänzend wird erwähnt, dass die oben erwähnten Schritte (ohne das Anbringen des Wirkstoffpflasters 3) der Desinfektion und Perforation an derselben Stelle wiederholt werden können, um eine stärkere Behandlung zu erzielen. Alternativ kann die Behandlung aber auch an beispielsweise direkt aneinander angrenzenden Hautstellen 1 wiederholt werden, um ein größeres Hautareal zu behandeln.

Weiterhin wird bei dem oben beschriebenen Ausführungsbeispiel das Wirkstoffpflaster 3 manuell von der Bedienperson auf die Hautstelle 1 aufgebracht. Ein derartiges Vorgehen ist dann zweckmäßig, wenn der Applikator 10 für unterschiedliche Wirkstoffpflaster 3 verwendet werden soll. In Abwandlung des oben beschriebenen Ausführungsbeispiels ist es darüber hinaus jedoch möglich, den Applikator 10 dahingehend auszubilden, dass in dem Applikator 10 analog zu den Funktionseinheiten 30 und 40 eine oder mehrere zusätzliche Funktionseinheiten angeordnet sind. Diese zusätzlichen Funktionseinheiten sind dann ebenso wie die Funktionseinheiten 30, 40 an der Trägereinrichtung 21 austausch- bzw. auswechselbar angeordnet. In jeder dieser zusätzlichen Funktionseinheiten können beispielsweise eine bestimmte Anzahl an Wirkstoffpflastern (unterschiedlichen Formats) gespeichert sein, die nach Aktivierung über die Steuereinrichtung 20 vollautomatisch auf die zuvor desinfizierte und perforierte Hautstelle 1 aufgebracht werden. In diesem Fall kann selbstverständlich auf eine Markiereinrichtung 47 verzichtet werden. Bei einem derart abgewandelten Applikator 10 werden somit alle Schritte vollautomatisch nach Aktivierung des Betätigungsschalters 19 (bzw. abgewandelter Bedienelemente, über die beispielsweise unterschiedliche Pflasterformate oder Behandlungsabläufe angewählt werden können) ausgelöst.

Weiterhin kann der Applikator 10 auch dahingehend abgewandelt werden, dass anstatt der Funktionseinheit 40 mit den Mikronadeln 42 bzw. dem Perforationsstempel 43 eine Funktionseinheit zum Einsatz kommt, die aus porösem Silizium oder biologisch abbaubarem Polymer bestehende Mikronadeln aufweist. Diese Mikronadeln werden durch eine geeignete Einrichtung nach Durchstechen der Haut von einem Träger abgetrennt und verbleiben danach in der Haut, bis die interzelluläre Flüssigkeit diese langsam von selbst auflöst.

Selbstverständlich kann der mechanische Aufbau des Applikators 10 stark von dem beschriebenen Ausführungsbeispiel abweichen. Insbesondere können hierbei die Anordnung und Ansteuerungen sowie auch die Ausbildungen der Funktionseinheiten 30, 40 zur Desinfektion und zur Perforation andersartig ausgebildet sein.

## Patentansprüche

1. Applikator (10) zur Behandlung von Haut, mit einem Gehäuse (11), in dem eine Einrichtung (40) zur Perforation einer Hautstelle (1) angeordnet ist, wobei die Einrichtung (40) zur Perforation der Haut durch eine Öffnung (16) im Gehäuse (11) in Kontakt mit der Hautstelle (1) anlegbar ist, wobei in dem Gehäuse (11) zusätzlich wenigstens eine Einrichtung (30) zur Desinfektion angeordnet ist, wobei die Einrichtung (30) zur Des-infektion durch dieselbe Öffnung (16) auf die Hautstelle (1) wirkt,
**dadurch gekennzeichnet, dass** eine Steuereinrichtung (20) vorgesehen ist, die die Einrichtung (40) zur Perforation und die Einrichtung (30) zur Desinfektion selbstständig entsprechend zumindest einen abgespeicherten Programmablaufs ansteuert und, dass Mittel vorgesehen sind, die die Einrichtung (40) zur Perforation und die Einrichtung (30) zur Desinfektion entsprechend des Programmablaufs durch die Öffnung (16) des Gehäuses (11) in Wirkverbindung mit der Hautstelle (1) bringen.

2. Applikator nach Anspruch 1,
**dadurch gekennzeichnet,**
**dass** zusätzlich eine Einrichtung zum Anbringen eines Wirkstoffpflasters auf die zuvor desinfizierte und perforierte Hautstelle (1) vorgesehen ist und, dass die Einrichtung zum Anbringen eines Wirkstoffpflasters (3) ebenfalls von der Steuereinrichtung (20) automatisch ansteuerbar ist.

3. Applikator nach einem der Ansprüche 1 oder 2,
**dadurch gekennzeichnet,**
**dass** die Einrichtung (30) zur Desinfektion einen mittels eines Desinfektionsmittels behandelten Träger (38) aufweist, welcher unter Anlagekontakt über die zu desinfizierende Hautstelle (1) bewegbar ist.

4. Applikator nach Anspruch 3,
**dadurch gekennzeichnet,**
**dass** der Träger (38) bandförmig ausgebildet ist, dass der Träger (38) zumindest auf seiner in Kontakt mit der Hautstelle (1) gelangenden Außenseite einen saugfähigen Stoff aufweist und, dass ein Vorratsbehälter (39) für das Desinfektionsmittel vorgesehen ist, über den die Außenseite des Trägers (38) mit Desinfektionsmittel benetzbar ist.

5. Applikator nach Anspruch 3 oder 4,
**dadurch gekennzeichnet,**
**dass** das Desinfektionsmittel eingefärbt ist und beim Kontakt mit der Hautstelle (1) diese farblich kennzeichnet.

6. Applikator nach einem der Ansprüche 1 bis 4,
**dadurch gekennzeichnet,**
**dass** eine Markiereinrichtung (47) vorgesehen ist, die die Einrichtung (30) zur Desinfektion bzw. die Einrichtung (40) zur Perforation umfangsseitig umgibt und die in Anlagekontakt mit der Hautstelle (1) bringbar ist, um die Hautstelle (1) bezüglich ihrer Ausdehnung optisch zu kennzeichnen.

7. Applikator nach einem der Ansprüche 1 bis 6,
**dadurch gekennzeichnet,**
**dass** die Einrichtung (40) zur Perforation einen Perforationsstempel (43) mit einer Vielzahl von nebeneinander angeordneten Mikronadeln (42) aufweist.

8. Applikator nach einem der Ansprüche 1 bis 6,
**dadurch gekennzeichnet,**
**dass** die Einrichtung (40) zur Perforation eine Vielzahl von Mikronadeln aufweist, die an einem Träger angeordnet sind und, dass die Mikronadeln als selbstauflösende Mikronadeln ausgebildet sind, welche von dem Träger abtrennbar sind.

9. Applikator nach einem der Ansprüche 1 bis 8,
**dadurch gekennzeichnet,**
**dass** in dem Gehäuse (11) eine Trägereinrichtung (21) zum Halten zumindest der Einrichtung (40) zur Perforation und der Einrichtung (30) zur Desinfektion angeordnet ist und, dass mittels der Trägereinrichtung (21) die Einrichtung (40) zur Perforation und die Einrichtung (30) zur Desinfektion in den Bereich der Öffnung (16) verschwenkbar sind.

10. Applikator nach Anspruch 9,
**dadurch gekennzeichnet,**
**dass** zumindest die Einrichtung (40) zur Perforation und der Einrichtung (30) zur Desinfektion an der Trägereinrichtung (21) auswechselbar angeordnet sind.

## Claims

1. Applicator (10) for treating skin, comprising a housing (11), in which a device (40) for perforating an area of skin (1) is arranged, which device (40) for perforating the skin can be brought into contact with the area of skin (1) through an opening (16) in the housing (11), wherein at least one disinfecting device (30) is additionally arranged in the housing (11), wherein the disinfecting device (30) acts on the area of skin (1) through the same opening (16), **characterized in that** a control device (20) is provided, which controls the perforating device (40) and the disinfecting device (30) independently in accordance with at least one stored program sequence, and **in that** means are provided which, in accordance with the program sequence, bring the perforating device (40) and the disinfecting device (30) into operative contact with the area of skin (1) through the opening (16) of the housing (11).

2. Applicator according to Claim 1, **characterized in that** a device for applying an active-substance patch to the previously disinfected and perforated area of skin (1) is additionally provided, and **in that** the device for applying an active-substance patch (3) can likewise be controlled automatically by the control device (20).

3. Applicator according to one of Claims 1 or 2, **characterized in that** the disinfecting device (30) has a carrier (38), which is treated by means of a disinfectant and which is movable with surface contact across the area of skin (1) to be disinfected.

4. Applicator according to Claim 3, **characterized in that** the carrier (38) is band-shaped, **in that** the carrier (38), at least on the outer face thereof coming into contact with the area of skin (1), has an absorbent material, and **in that** a reservoir (39) for the disinfectant is provided, via which reservoir (39) the outer face of the carrier (38) can be wetted with disinfectant.

5. Applicator according to Claim 3 or 4, **characterized in that** the disinfectant is dyed and, upon contact with the area of skin (1), identifies the latter by color.

6. Applicator according to one of Claims 1 to 4, **characterized in that** a marking device (47) is provided, which peripherally surrounds the disinfecting device (30) and/or the perforating device (40) and can be brought into surface contact with the area of skin (1) in order to visually identify the extent of the area of skin (1).

7. Applicator according to one of Claims 1 to 6, **characterized in that** the perforating device (40) has a perforating punch (43) with a multiplicity of micro-needles (42) arranged alongside one another.

8. Applicator according to one of Claims 1 to 6, **characterized in that** the perforating device (40) has a multiplicity of micro-needles, which are arranged on a carrier, and **in that** the micro-needles are designed as self-disintegrating micro-needles, which are detachable from the carrier.

9. Applicator according to one of Claims 1 to 8, **characterized in that** a support device (21) for holding at least the perforating device (40) and the disinfecting device (30) is arranged in the housing (11), and **in that** the perforating device (40) and the disinfecting device (30) can be pivoted into the area of the opening (16) by means of the support device (21).

10. Applicator according to Claim 9, **characterized in that** at least the perforating device (40) and the disinfecting device (30) are arranged exchangeably on the support device (21).

## Revendications

1. Applicateur (10) pour traitement de la peau, avec un boîtier (11) dans lequel est disposé un dispositif (40) de perforation d'un point cutané (1), le dispositif (40) de perforation de la peau étant placé en contact avec le point cutané (1) par une ouverture (16) pratiquée dans le boîtier (11), au moins un dispositif (30) de désinfection étant en outre disposé dans le boîtier (11), le dispositif (30) de désinfection agissant sur le point cutané (1) au travers de cette même ouverture (16), **caractérisé en ce qu'**un dispositif de commande (20) est prévu commandant le dispositif (40) de perforation et le dispositif (30) de désinfection automatiquement en fonction d'au moins une séquence de programme mémorisée et que des moyens sont prévus pour amener le dispositif (40) de perforation et le dispositif (30) de désinfection en liaison active avec le point cutané (1) au travers de l'ouverture (16) du boîtier (11) en fonction de la séquence de programme.

2. Applicateur selon la revendication 1, **caractérisé en ce qu'**un dispositif d'application d'un pansement de substance active sur le point cutané (1) précédemment désinfecté et perforé est en outre prévu et que le dispositif d'application d'un pansement de substance active (3) peut également être commandé automatiquement par le dispositif de commande (20).

3. Applicateur selon l'une quelconque des revendications 1 ou 2, **caractérisé en ce que** le dispositif (30) de désinfection comporte un support (38) traité à l'aide d'un moyen de désinfection pouvant être déplacé en contact d'appui au-delà du point cutané (1) à désinfecter.

4. Applicateur selon la revendication 3, **caractérisé en ce que** le support (38) prend une forme de bande, que le support (38) comporte au moins sur son côté extérieur entrant en contact avec le point cutané (1) une substance aspirable et qu'un bac de réserve (39) via lequel le côté extérieur du support (38) peut être humecté de moyen de désinfection est prévu pour le moyen de désinfection.

5. Applicateur selon la revendication 3 ou 4, **caractérisé en ce que** le moyen de désinfection est coloré et qu'il colore le point cutané (1) après avoir été en contact avec lui.

6. Applicateur selon l'une quelconque des revendications 1 à 4, **caractérisé en ce qu'**un dispositif de marquage (47) est prévu entourant le dispositif (30) de désinfection et/ou le dispositif (40) de perforation du côté périphérique et pouvant être amené en contact d'appui avec le point cutané (1) pour caractériser optiquement le point cutané (1) par rapport à son extension.

7. Applicateur selon l'une quelconque des revendications 1 à 6, **caractérisé en ce que** le dispositif (40) de perforation comporte un tampon perforateur (43) pourvu d'une pluralité de microaiguilles (42) disposées côte à côte.

8. Applicateur selon l'une quelconque des revendications 1 à 6, **caractérisé en ce que** le dispositif (40) de perforation comporte une pluralité de microaiguilles disposées au niveau d'un support et que les microaiguilles prennent la forme de microaiguilles autodéclenchables pouvant se détacher du support.

9. Applicateur selon l'une quelconque des revendications 1 à 8, **caractérisé en ce qu'**un dispositif de support (21) est disposé dans le boîtier (11) pour maintenir au moins le dispositif (40) de perforation et le dispositif (30) de désinfection et que le dispositif de support (21) permet de pivoter le dispositif (40) de perforation et le dispositif (30) de désinfection dans la région de l'ouverture (16).

10. Applicateur selon la revendication 9, **caractérisé en ce qu'**au moins le dispositif (40) de perforation et le dispositif (30) de désinfection sont disposés de façon remplaçable au niveau du dispositif de support (21).
